# EUROPEAN PATENT SPECIFICATION

(11) **EP 4 519 257 B1**
(45) Date of publication and mention of the grant of the patent: **29.07.2026**
(21) Application number: 24740590.5
(22) Date of filing: 20.06.2024
(51) Int. Cl.: C07D 413/12

(54) **PROCESS FOR THE PREPARATION OF PYROXASULFONE**
VERFAHREN ZUR HERSTELLUNG VON PYROXASULFON
PROCÉDÉ DE PRÉPARATION DE PYROXASULFONE

(30) Priority: 22.06.2023 EP 23181049; 29.11.2023 EP 23212931; 01.02.2024 EP 24155335
(43) Date of publication of application: 12.03.2025
(73) Proprietor: Adama Agan Ltd., 7710201 Ashdod (IL)
(72) Inventor: COHEN, Yair, 7685925 Mazkeret Batya (IL); FRONTON, Sveta, 7174650 Modiin (IL); ASHUSH, Natali, 4284200 Bat Hefer (IL)
(74) Representative: Modiano, Gabriella Diana
(86) International application number: PCT/IL2024/050604
(87) International publication number: WO 2024/261759

(56) References cited:
- EP-A2- 4 053 125
- WO-A1-2023/047416

## Description

### FIELD OF THE INVENTION

The present invention relates to the field of synthesis of organic compounds, more specifically to a process for the preparation of pyroxasulfone.

### BACKGROUND PRIOR ART

US2023012374 discloses the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) by oxidizing 3-[(5-difluoromethoxy-1-methyl-3-trifluoromethylpyrazol-4-yl)methylthio]-4,5-dihydro-5,5-dimethylisoxazole in the presence of a metal catalyst and hydrogen peroxide. The yields of the reaction are typically lowered by incomplete oxidation of the thio group to the corresponding sulfoxide. Different organic solvents are tested in US2023012374, for example, butyl acetate, isopropyl acetate, ethyl acetate, methanol, butanol, ethanol, acetonitrile, DMF, or NMP, each solvent and solvent amount having a different effect on the amount pyroxasulfone formed and the resulting amounts of the sulfoxide intermediate impurity. According to the tested organic solvents in US2023012374, the amount of organic solvent ranges from 0.4 to 1.8 liters of organic solvent per mole of 3-[(5-difluoromethoxy-1-methyl-3-trifluoromethylpyrazol-4-yl)methylthio]-4,5-dihydro-5,5-dimethylisoxazole.

### SUMMARY OF THE INVENTION

According to an embodiment of this invention, conducting the oxidation in the presence of at least one carbonate ester as solvent greatly reduces the sulfoxide impurity. According to another embodiment of the invention, the amount of the at least one carbonate ester solvent necessary for the reaction is significantly reduced relative to the solvent amount required by US2023012374. Also, as consequence of this reduction in the amount of solvent, a higher load of reactants can be achieved for the same volume, and therefore, a higher quantity of pyroxasulfone product can be achieved in each production batch for any given equipment, relative to the amounts obtained by US2023012374.

Thus, the present application is directed to a process for the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst, wherein the solvent comprises at least one C₁-C₂₄ carbonate ester and water.

The reaction can be summarized in the following equation:

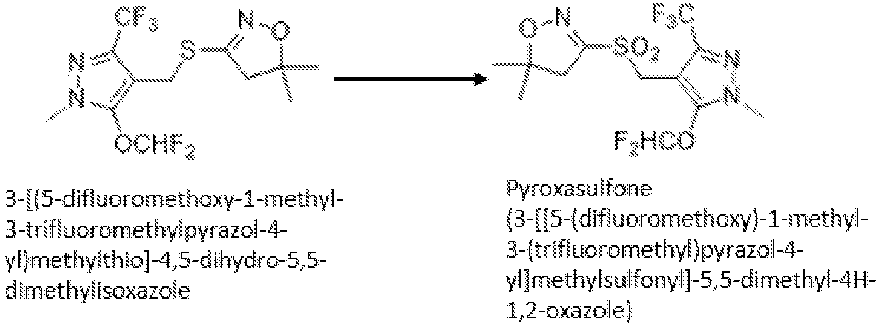

### DETAILED DESCRIPTION OF THE INVENTION

### Definitions

Embodiments of the present invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. While a number of embodiments and features are described herein, it is to be understood that the various features of the invention and aspects of embodiments, even if described separately, may be combined unless mutually exclusive or contrary to the specific description. All references cited herein are incorporated by reference as if each had been individually incorporated.

As used herein, the transitional term "comprising" or "that comprises", which is synonymous with "including," or "containing," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

The term "C₁-C₁₂ alkyl" means a linear or branched saturated hydrocarbon chain radical having no multiple bonds and having from one to twelve carbon atoms, and which is attached to the rest of the molecule by a single bond. Suitable groups include, but are not limited to alkyl groups such as methyl, ethyl, propyl (e.g. n-propyl or iso-propyl), butyl (e.g. n-butyl, t-butyl, sec-butyl), pentyl (e.g. 1-methylpentyl, 3-methylpentyl, n-pentyl), hexyl, octyl, or dodecyl.

The term "C₂-C₁₂ alkenyl" means a linear or branched hydrocarbon chain radical having one or more carbon-carbon double bonds therein and having from two to twelve carbon atoms, and which is attached to the rest of the molecule by a single bond. The double bond of an alkenyl group can be unconjugated or conjugated to another unsaturated group. Suitable alkenyl groups include, but are not limited to alkenyl groups such as vinyl, allyl, butenyl (e.g. 1-butenyl, 2-butenyl, 3-butenyl), pentenyl (e.g. 1-pentenyl, 2-pentenyl, 3-pentenyl, 4-pentenyl,), hexenyl (e.g. 1-hexenyl, 2-hexenyl, 3-hexenyl, 4-hexenyl, 5-hexenyl,), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl,), 2-propyl-2-butenyl, 4,6-Dimethyl-oct-6-enyl.

The term "C₂-C₁₂ alkynyl" means a linear or branched hydrocarbon chain radical having one or more carbon-carbon triple bonds therein and from two to twelve carbon atoms, and which is attached to the rest of the molecule by a single bond. The triple bond of an alkynyl group can be unconjugated or conjugated to another unsaturated group. Suitable alkynyl groups include, but are not limited to alkynyl groups such as ethynyl, propynyl (e.g. 1-propynyl, 2-propynyl), butynyl (e.g. 1-butynyl, 2-butynyl, 3-butynyl), pentynyl (e.g. 1-pentynyl, 2-pentynyl, 3-pentynyl, 4-pentynyl,), hexynyl (e.g. 1-hexynyl, 2-hexynyl, 3-hexynyl, 4-hexynyl, 5-hexynyl,), methylpropynyl, 3-methyl-1-butynyl, 4-methyl-2-heptynyl , and 4-ethyl-2-octynyl.

"C₆-C₁₅ Aryl" refers to a hydrocarbon moiety having six to fifteen carbon atoms and at least one aromatic hydrocarbon structure, such as phenyl, naphthyl or anthracyl.

"C₇-C₂₄ Arylalkyl" refers to an aryl group linked to the rest of the molecule by an alkyl group, such as benzyl and phenethyl.

In each case, the groups "C₁-C₁₂ alkylene", "C₁-C₁₂ alkenylene", "C₁-C₁₂ alkynylene", "C₆-C₁₅ arylene", and "C₇-C₁₅ arylalkylene" have the same meaning as "C₁-C₁₂ alkyl", "C₁-C₁₂ alkenyl", "C₁-C₁₂ alkynyl", "C₆-C₁₅ aryl", and "C₇-C₂₄ arylalkyl", respectively, but wherein the groups are attached to the rest of the molecule by two single bonds. For example, where ethyl represent "CH₃-CH₂-", ethylene represents -CH₂-CH₂-, or where propyl represents CH₃-CH₂-CH₂-, propylene represents - CH₂-CH₂-CH₂- or -CH₂-CH(CH₃)-.

"C₁-C₂₄ carbonate ester" refers in the present application to a carbonate ester of formula (I) wherein C₁-C₂₄ refers to each of R¹ and R² attached to the acyl group and it is selected from the group consisting of C₁-C₁₂ alkyl, C₁-C₁₂ alkenyl, C₁-C₁₂ alkynyl, C₆-C₁₅ aryl, and C₇-C₂₄ arylalkyl; or both R¹ and R² together form a group selected from C₁-C₁₂ alkylene, C₁-C₁₂ alkenylene, C₁-C₁₂ alkynylene, C₆-C₁₅ arylene, and C₇-C₁₅ arylalkylene.

The term "a" or "an" as used herein includes the singular and the plural, unless specifically stated otherwise. Therefore, the terms "a," "an" or "at least one" can be used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±10% from the indicated values in the range. In addition, the endpoints of all ranges directed to the same component or property herein are inclusive of the endpoints, are independently combinable, and include all intermediate points and ranges. Similarly, the ranges and amounts for each element of the technology described herein can be used together with ranges or amounts for any of the other elements.

### Process for the preparation of pyroxasulfone

In a first aspect, the present disclosure relates to a process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst, wherein the solvent comprises at least one C₁-C₂₄ carbonate ester and water.

This process provides an alternative to the existing processes for the preparation of pyroxasulfone, and particularly, it provides a process wherein the amount of organic solvent, i.e. carbonate ester, is lower than the amount of organic solvent when the solvent is an alcohol, nitrile, carboxylic acid ester or amide.

The at least one C₁-C₂₄ carbonate ester may be a compound of formula (I) 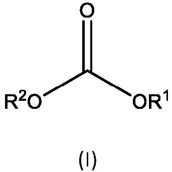
wherein each of R¹ and R² is selected from the group consisting of C₁-C₁₂ alkyl, C₁-C₁₂ alkenyl, C₁-C₁₂ alkynyl, C₆-C₁₅ aryl, and C₇-C₂₄ arylalkyl; or
both R¹ and R² together form a group selected from C₁-C₁₂ alkylene, C₁-C₁₂ alkenylene, C₁-C₁₂ alkynylene, C₆-C₁₅ arylene, and C₇-C₁₅ arylalkylene.

The at least one C₁-C₂₄ carbonate ester may be a compound of formula (I) wherein each of R¹ and R² is selected from the group consisting of C₁-C₁₂ alkyl.

The at least one C₁-C₂₄ carbonate ester may be a compound of formula (I) wherein each of R¹ and R² is selected from the group consisting of C₁-C₄ alkyl.

The at least one C₁-C₂₄ carbonate ester may be a compound of formula (I) wherein R¹ and R² are both methyl or ethyl.

The at least one C₁-C₂₄ carbonate ester may be a compound of formula (I) wherein R¹ and R² together are -(CH₂-CH₂)- or -(CH(CH)₃)CH₂)-.

The at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, n-butyl propyl carbonate, ethylene carbonate, propylene carbonate, 1,2-butylene carbonate, trans-2,3-butylene carbonate, or trimethylene carbonate. The at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate, or trans-2,3-butylene carbonate. The at least one C₁-C₂₄ carbonate ester may be selected from the group consisting of dimethyl carbonate, ethyl methyl carbonate, methyl propyl carbonate, n-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate or trans-2,3-butylene carbonate. When a combination of two or more C₁-C₂₄ carbonate esters is used, the ratio of the two or more C₁-C₂₄ carbonate ester may be any ratio as long as the process proceeds.

The amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole.

In one embodiment, the at least one C₁-C₂₄ carbonate ester may be continuously distilled out during the reaction and fresh C₁-C₂₄ carbonate ester may be added, with the proviso that the amount of the at least one C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole is maintained within the hereinabove mentioned ranges.

The amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² is selected from the group consisting of C₁-C₁₂ alkyl or a compound of formula (I) wherein R¹ and R² together are a C₁-C₁₂ alkylene. The amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² is selected from the group consisting of C₁-C₄ alkyl or a compound of formula (I) wherein R¹ and R² together are a C₁-C₄ alkylene. The amount of the at least one C₁-C₂₄ carbonate ester may range from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² are both methyl or ethyl or a compound of formula (I) wherein R¹ and R² together are -(CH₂-CH₂)- or - (CH(CH)₃)CH₂)-.

The amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, methyl n-pentyl carbonate, diethyl carbonate, ethyl propyl carbonate, n-butyl ethyl carbonate, tert-butyl ethyl carbonate, ethyl pentyl carbonate, dipropyl carbonate, diisopropyl carbonate, dibutyl carbonate, n-butyl propyl carbonate, dioctyl carbonate, divinyl carbonate, diallyl carbonate, allyl methyl carbonate, diphenyl carbonate, methyl phenyl carbonate, ethyl phenyl carbonate, phenyl propyl carbonate, tert-butyl phenyl carbonate, benzyl methyl carbonate, benzyl ethyl carbonate, benzyl vinyl carbonate, benzyl phenyl carbonate, ethylene carbonate, propylene carbonate, 1,2-butylene carbonate, trans-2,3-butylene carbonate, trimethylene carbonate or vinylene carbonate. The amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, diallyl carbonate, allyl methyl carbonate, diphenyl carbonate, methyl phenyl carbonate, ethyl phenyl carbonate, benzyl methyl carbonate, benzyl vinyl carbonate, benzyl phenyl carbonate, ethylene carbonate, propylene carbonate, 1,2-butylene carbonate, trans-2,3-butylene carbonate or vinylene carbonate. The amount of the at least one C₁-C₂₄ carbonate ester may range from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate or trans-2,3-butylene carbonate. The amount of the at least one C₁-C₂₄ carbonate ester may range from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate or trans-2,3-butylene carbonate.

The amount of water may range from 0.01 to 1, from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole. The amount of water in the process of the present invention includes water added as solvent in the reaction vessel and water included in the aqueous hydrogen peroxide solution.

The amount of water may range from 0.01 to 1, from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole and the amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole. The amount of water may range from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole and the amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole. The amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole and the amount of the at least one C₁-C₂₄ carbonate ester may range from 0.05 to 0.35, or from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole.

The amount of water may range from 0.01 to 1, from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² is selected from the group consisting of C₁-C₁₂ alkyl or a compound of formula (I) wherein R¹ and R² together are a C₁-C₁₂ alkylene. The amount of water may range from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² is selected from the group consisting of C₁-C₄ alkyl or a compound of formula (I) wherein R¹ and R² together are a C₁-C₄ alkylene. The amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² are both methyl or ethyl or a compound of formula (I) wherein R¹ and R² together are -(CH₂-CH₂)- or -(CH(CH)₃)CH₂)-.

The amount of water may range from 0.01 to 1, from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, methyl n-pentyl carbonate, diethyl carbonate, ethyl propyl carbonate, n-butyl ethyl carbonate, tert-butyl ethyl carbonate, ethyl pentyl carbonate, dipropyl carbonate, diisopropyl carbonate, dibutyl carbonate, n-butyl propyl carbonate, dioctyl carbonate, divinyl carbonate, diallyl carbonate, allyl methyl carbonate, diphenyl carbonate, methyl phenyl carbonate, ethyl phenyl carbonate, phenyl propyl carbonate, tert-butyl phenyl carbonate, benzyl methyl carbonate, benzyl ethyl carbonate, benzyl vinyl carbonate, benzyl phenyl carbonate, ethylene carbonate, propylene carbonate, 1,2-butylene carbonate, trans-2,3-butylene carbonate, trimethylene carbonate or vinylene carbonate. The amount of water may range from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, diallyl carbonate, allyl methyl carbonate, diphenyl carbonate, methyl phenyl carbonate, ethyl phenyl carbonate, benzyl methyl carbonate, benzyl vinyl carbonate, benzyl phenyl carbonate, ethylene carbonate, propylene carbonate, 1,2-butylene carbonate, trans-2,3-butylene carbonate or vinylene carbonate. The amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate or trans-2,3-butylene carbonate. The amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate or trans-2,3-butylene carbonate.

The ratio of the at least one C₁-C₂₄ carbonate ester to water may range from 90:10 to 30:70, from 80:20 to 50:50, from 75:25 to 60:40 by volume ratio.

The ratio of the at least one C₁-C₂₄ carbonate ester to water may range from 90:10 to 30:70, from 80:20 to 50:50, from 75:25 to 60:40 by volume ratio, the amount of water may range from 0.01 to 1, from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole and the amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole. The ratio of the at least one C₁-C₂₄ carbonate ester to water may range from 80:20 to 50:50, from 75:25 to 60:40 by volume ratio, the amount of water may range from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole and the amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole. The ratio of the at least one C₁-C₂₄ carbonate ester to water may range from 75:25 to 60:40 by volume ratio, the amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole and the amount of the at least one C₁-C₂₄ carbonate ester may range from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole.

The ratio of the at least one C₁-C₂₄ carbonate ester to water may range from 90:10 to 30:70, from 80:20 to 50:50, from 75:25 to 60:40 by volume ratio, the amount of water may range from 0.01 to 1, from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² is selected from the group consisting of C₁-C₁₂ alkyl or a compound of formula (I) wherein R¹ and R² together are a C₁-C₁₂ alkylene. The ratio of the at least one C₁-C₂₄ carbonate ester to water may range from 80:20 to 50:50, from 75:25 to 60:40 by volume ratio, the amount of water may range from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² is selected from the group consisting of C₁-C₄ alkyl or a compound of formula (I) wherein R¹ and R² together are a C₁-C₄ alkylene. The ratio of the at least one C₁-C₂₄ carbonate ester to water may range from 75:25 to 60:40 by volume ratio, the amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be a compound of formula (I) wherein each of R¹ and R² are both methyl or ethyl or a compound of formula (I) wherein R¹ and R² together are -(CH₂-CH₂)- or -(CH(CH)₃)CH₂)-.

The ratio of the at least one C₁-C₂₄ carbonate ester to water may range from 90:10 to 30:70, from 80:20 to 50:50, from 75:25 to 60:40 by volume ratio, the amount of water may range from 0.01 to 1, from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 1.25, from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, methyl n-pentyl carbonate, diethyl carbonate, ethyl propyl carbonate, n-butyl ethyl carbonate, tert-butyl ethyl carbonate, ethyl pentyl carbonate, dipropyl carbonate, diisopropyl carbonate, dibutyl carbonate, n-butyl propyl carbonate, dioctyl carbonate, divinyl carbonate, diallyl carbonate, allyl methyl carbonate, diphenyl carbonate, methyl phenyl carbonate, ethyl phenyl carbonate, phenyl propyl carbonate, tert-butyl phenyl carbonate, benzyl methyl carbonate, benzyl ethyl carbonate, benzyl vinyl carbonate, benzyl phenyl carbonate, ethylene carbonate, propylene carbonate, 1,2-butylene carbonate, trans-2,3-butylene carbonate, trimethylene carbonate or vinylene carbonate. The ratio of the at least one C₁-C₂₄ carbonate ester to water may range from 80:20 to 50:50, from 75:25 to 60:40 by volume ratio, the amount of water may range from 0.05 to 1, from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.75, from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, tert-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, diallyl carbonate, allyl methyl carbonate, diphenyl carbonate, methyl phenyl carbonate, ethyl phenyl carbonate, benzyl methyl carbonate, benzyl vinyl carbonate, benzyl phenyl carbonate, ethylene carbonate, propylene carbonate, 1,2-butylene carbonate, trans-2,3-butylene carbonate or vinylene carbonate. The ratio of the at least one C₁-C₂₄ carbonate ester to water may range from 75:25 to 60:40 by volume ratio, the amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.35, from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate or trans-2,3-butylene carbonate. The ratio of the at least one C₁-C₂₄ carbonate ester to water may range from 75:25 to 60:40 by volume ratio, the amount of water may range from 0.1 to 0.8 liters per mole of 3-[(5-difluoromethoxy-1-methyl-3-trifluoromethylpyrazol-4-yl)methylthio]-4,5-dihydro-5,5-dimethylisoxazole, the amount of the at least one C₁-C₂₄ carbonate ester may range from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole and the at least one C₁-C₂₄ carbonate ester may be selected from the group consisting of dimethyl carbonate, ethyl methyl carbonate, methyl propyl carbonate, n-butyl methyl carbonate, diethyl carbonate, ethyl propyl carbonate, n-butyl ethyl carbonate, tert-butyl ethyl carbonate, dipropyl carbonate, diisopropyl carbonate, dibutyl carbonate, ethylene carbonate, propylene carbonate, 1,2-butylene carbonate or trans-2,3-butylene carbonate.

The hydrogen peroxide in the process of the invention may be a 10 to 70 wt %, 20 to 65%, 25% to 60% aqueous hydrogen peroxide solution.

The amount of hydrogen peroxide may range from 2 to 8, from 2 to 6, from 2 to 3 moles per mole of 3-[(5-difluoromethoxy-1-methyl-3-trifluoromethylpyrazol-4-yl)methylthio]-4,5-dihydro-5,5-dimethylisoxazole.

The amount of hydrogen peroxide may range from 2 to 8, from 2 to 6, from 2 to 3 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 10 to 70 wt %, 20 to 65%, 25% to 60% aqueous hydrogen peroxide solution. The amount of hydrogen peroxide may range from 2 to 6, from 2 to 3 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 20 to 65%, 25% to 60% aqueous hydrogen peroxide solution. The amount of hydrogen peroxide may range from 2 to 3 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 25% to 60% aqueous hydrogen peroxide solution.

The metal catalyst may be selected from the groups 5 to 6 of the periodic table, selected from a tungsten catalyst, a molybdenum catalyst and a niobium catalyst. The tungsten catalyst may be selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture thereof. The tungsten catalyst may be selected from the group of tungstic acid, alkali or alkaline earth salt of tungstic acid, metal tungsten, tungsten oxide, tungsten carbide, or a mixture thereof. The molybdenum catalyst may be selected from the group of molybdic acid, molybdic acid salt, metal molybdenum, molybdenum oxide, molybdenum carbide, molybdenum chloride, or a mixture thereof. The niobium catalyst may be selected from the group of niobic acid, niobic acid salt, metal niobium, niobium oxide, niobium carbide, niobium chloride, or a mixture thereof. The metal catalyst may be sodium tungstate dihydrate or ammonium molybdate tetrahydrate.

The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole.

The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole and the metal catalyst may be selected from the groups 5 to 10, from the groups 5 to 6 of the periodic table, selected from a tungsten catalyst, a molybdenum catalyst and a niobium catalyst. The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole and the metal catalyst may be selected from the groups 5 to 6 of the periodic table, selected from a tungsten catalyst, a molybdenum catalyst and a niobium catalyst. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole and the metal catalyst may be selected from a tungsten catalyst, a molybdenum catalyst and a niobium catalyst. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole and the metal catalyst may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture thereof. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole and the metal catalyst may be sodium tungstate dihydrate or ammonium molybdate tetrahydrate.

The temperature of the reaction in the process of the present invention may range from 30°C to 120°C, from 40°C to 100°C, from 50°C to 95°C.

The reaction in the process of the present invention may be performed in a time that ranges from 1 hour to 24 hours, from 2 hours to 16 hours, from 3 hours to 12 hours.

The reaction in the process of the present invention may be performed in a time that ranges from 1 hour to 24 hours, from 2 hours to 16 hours, from 3 hours to 12 hours and the temperature of the reaction may range from 30°C to 120°C, from 40°C to 100°C, from 50°C to 95°C. The reaction in the process of the present invention may be performed in a time that ranges from 2 hours to 16 hours, from 3 hours to 12 hours and the temperature of the reaction may range from 40°C to 100°C, from 50°C to 95°C. The reaction in the process of the present invention may be performed in a time that ranges from 3 hours to 12 hours and the temperature of the reaction may range from 50°C to 95°C.

The process of the present invention may further comprise a phase transfer catalyst. The process of the present invention may further comprise a phase transfer catalyst selected from the group of tetrabutylammonium chloride, tetrabutylammonium bromide, tetrabutylammonium hydrogen sulfate, tetraoctyl ammonium bromide, tetraoctyl ammonium bromide, methyltrialkyl ammonium chloride (Adogen 464), tetradecyltrimethyl ammonium bromide, methyltrioctyl ammonium chloride (Aliquat 336), didecyldimethyl ammonium chloride, benzyltrimethyl ammonium chloride, benzyltriethyl ammonium chloride, octyltrimethyl ammonium chloride, octyltrimethyl ammonium bromide, trioctylmethyl ammonium chloride, trioctylmethyl ammonium bromide, tetrabutyl phosphonium bromide, tetraoctyl phosphonium bromide or tetraphenyl phosphonium bromide.

The amount of the further phase transfer catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole.

The process of the present invention may further comprise an acid catalyst. The process of the present invention may further comprise an acid catalyst selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate.

The amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole.

The amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The metal catalyst may be selected from the groups 5 to 10, from the groups 5 to 6 of the periodic table, selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The metal catalyst may be selected from the groups 5 to 6 of the periodic table, selected from a tungsten catalyst, a molybdenum catalyst and a niobium catalyst, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The metal catalyst may be selected from a tungsten catalyst, a molybdenum catalyst and a niobium catalyst, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst may be selected from the groups 5 to 10, from the groups 5 to 6 of the periodic table, selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst may be selected from the groups 5 to 6 of the periodic table, selected from a tungsten catalyst, a molybdenum catalyst and a niobium catalyst, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst may be selected from a tungsten catalyst, a molybdenum catalyst and a niobium catalyst, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.The process of the present invention may comprise the following steps: i) addition of 3-[(5-difluoromethoxy-1-methyl-3-trifluoromethylpyrazol-4-yl)methylthio]-4,5-dihydro-5,5-dimethylisoxazole, the at least one C₁-C₂₄ carbonate ester, and optional addition of the metal catalyst, water and organic solvent to the reaction vessel; ii) addition of the aqueous hydrogen peroxide solution; iii) optional addition of the metal catalyst, water and organic solvent; iv) isolation and purification of the obtained pyroxasulfone from the reaction mixture, with the proviso that the metal catalyst is added at least in one of the steps i) or iii).

The metal catalyst may be added to the reaction vessel before and/or after the addition of the aqueous hydrogen peroxide solution. Water as solvent may be added to the reaction vessel before and/or after the addition of the aqueous hydrogen peroxide solution, or it may not be added to the reaction vessel. The organic solvent may be added to the reaction vessel before and/or after the addition of the aqueous hydrogen peroxide solution, or it may not be added to the reaction vessel.

The metal catalyst may be added to the reaction vessel as solid or pre-dissolved in water or in the organic solvent, it may be added in one, two, or several portions, or it may be added in continuous way.

The aqueous hydrogen peroxide solution may be added at once, in several portions, or it may be added dropwise.

Previously to the isolation and purification of the obtained pyroxasulfone from the reaction mixture, any possible unreacted hydrogen peroxide may be decomposed by treating the reaction mixture with any reducing agent known by the person skilled in the art, like for example and in a not limiting way, sodium sulfite.

The isolation and purification of pyroxasulfone may be performed by any method known by the person skilled in the art, like for example and in a not limiting way, extraction, washing, or crystallization.

A second aspect of the invention is the use of pyroxasulfone obtained according to the process hereinabove disclosed in the preparation of an agrochemical composition.

### Examples

### Dimethyl carbonate (DMC)

3-[(5-difluoromethoxy-1-methyl-3-trifluoromethylpyrazol-4-yl)methylthio]-4,5-dihydro-5,5-dimethylisoxazole (100 gr, 78 % purity) is added to a 1-liter reactor followed by 200 ml of dimethyl carbonate (DMC) and stirring is started. The mixture is heated to 55 °C. A solution of catalyst is prepared as follows: Sodium tungstate hydrate (0.02 eq. 1.42 gr) is dissolved in 10 ml of water. Half of the catalyst solution (5.7 gr) is added to the reactor. Then, the reaction is fed with hydrogen peroxide (28-30%, 4.2 eq., 103.36 gr) during 2 hrs. At the beginning of the addition a slight exotherm is observed, and after it's detraction the temperature is increased to 90 °C. After the addition of hydrogen peroxide, the second half of the catalyst solution is added. After aging of additional 4 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.3% (230 nm). Then 100ml of MeOH are added followed by the addition of 100ml of water. The mixture is heated to 82-85 °C for 1 hour. Then the mixture is cooled to 15°C along 2 hours.

The mixture is filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 90.87%

### Diethyl carbonate (DEC)

3-[(5-difluoromethoxy-1-methyl-3-trifluoromethylpyrazol-4-yl)methylthio]-4,5-dihydro-5,5-dimethylisoxazole (100 gr, 78 % purity) is added to a 1-liter reactor followed by 100 ml of diethyl carbonate (DEC) and stirring is started. The mixture is heated to 55 °C. A solution of catalyst is prepared as follows: sodium tungstate hydrate (0.02 eq. 1.42 gr) is dissolved in 10 ml of water. Half of the catalyst solution (5.7 gr) is added to the reactor. Then, the reaction is fed with hydrogen peroxide (28-30%, 4.2 eq., 103.36 gr) during 2 hrs. At the beginning of the addition a slight exotherm is observed, and after it's detraction the temperature is increased to 90 °C.After the addition of hydrogen peroxide, the second half of the catalyst solution is added. After aging of additional 6 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.31% (230 nm). Then 100ml of MeOH are added followed by the addition of 100ml of water. The mixture is heated to 82-85 °C for 1 hour. Then the mixture is cooled to 15°C along 2 hours.

The mixture is filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 87.49%

### Propylene carbonate (PC)

3-[(5-difluoromethoxy-1-methyl-3-trifluoromethylpyrazol-4-yl)methylthio]-4,5-dihydro-5,5-dimethylisoxazole (100 gr, ~78 % purity) is added to a 1-liter reactor followed by 100 ml of propylene carbonate (PC) and stirring is started. The mixture is heated to 55 °C. A solution of catalyst is prepared as follows: sodium tungstate hydrate (0.02 eq. 1.42 gr) is dissolved in 10 ml of waterHalf of the catalyst solution (5.7 gr) is added to the reactor. Then, the reaction is fed with hydrogen peroxide (28-30%, 4.2 eq., 103.36 gr) during 2 hrs. At the beginning of the addition a slight exotherm is observed, and after it's detraction the temperature is increased to 90 °C. After the addition of hydrogen peroxide, the second half of the catalyst solution is added. After aging of additional 4 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.05% (230 nm). Then 100ml of MeOH are added followed by the addition of 100ml of water. The mixture is heated to 82-85 °C for 1 hour. Then the mixture is cooled to 15°C along 2 hours.

The mixture was filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 88.48%

### Ethylene carbonate (EC)

3-[(5-difluoromethoxy-1-methyl-3-trifluoromethylpyrazol-4-yl)methylthio]-4,5-dihydro-5,5-dimethylisoxazole (100 gr, 78 % purity) is added to a 1-liter reactor followed by 100 ml of Ethylene carbonate (EC) and stirring is started. The mixture is heated to 55 °C. A solution of catalyst is prepared as follows: sodium tungstate hydrate (0.02 eq. 1.42 gr) is dissolved in 10 ml of water. Half of the catalyst solution (5.7 gr) is added to the reactor. Then, the reaction is fed with hydrogen peroxide (28-30%, 4.2 eq., 103.36 gr) during 2 hrs. At the beginning of the addition a slight exotherm is observed, and after it's detraction the temperature is increased to 90 °C. After the addition of hydrogen peroxide, the second half of the catalyst solution is added. After aging of additional 2 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.19% (230 nm). Then 100ml of MeOH are added followed by the addition of 100ml of water. The mixture is heated to 82-85 °C for 1 hour. Then the mixture is cooled to 15°C along 2 hours.

The mixture is filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 84%.

### Dimethyl carbonate (DMC)

3-[(5-difluoromethoxy-1-methyl-3-trifluoromethylpyrazol-4-yl)methylthio]-4,5-dihydro-5,5-dimethylisoxazole (200 gr, 75 % purity) is added to a 1-liter reactor followed by 100 ml of dimethyl carbonate (DMC) and stirring is started. The mixture is heated to 80 °C. A solution of catalyst is prepared as follow: Sodium tungstate hydrate (0.02 eq. 2.8 gr) is dissolved in 10 ml of water. The catalyst solution is added to the reactor followed by addition of sulfuric acid (0.02eq, 0.83 gr, 98%). Then, the reaction is fed with hydrogen peroxide (28-30%, 4.2 eq., 199 gr) during 2 hrs. At the beginning of the addition a slight exotherm is observed, and after its detraction the temperature is increased to 80-90 °C. Then, after additional 6 hours of reaction, the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.08% (230 nm). Then, 100 ml of water are added to the reaction. The mixture is heated to 80 °C for 0.5 hour. Then, the mixture is cooled to 15°C for 2 hours. The mixture is filtered, the resulting precipitate is washed with cold MeOH (twice 100ml), NaHCO₃ (5%, 100 ml) and water (twice 100ml), and dried over night at 50 °C under vacuum, to obtain pyroxasulfone with a yield of 90%.

### Comparative examples with methanol (MeOH)

Example 2-12 from the prior art document US2023012374 is repeated under the same conditions of temperature and reaction time, but with the following quantities:

| Example | M359 | MeOH | Added water | Na2WO4·2H2O | H₂O₂ 30% aq. solution | H₂SO₄ | % M375 |
|---|---|---|---|---|---|---|---|
| Comparative 1 | 23 gr | 19.1 ml | 76.5 ml | 0.42 gr | 36.3 gr | 0.63 gr | 48.04% |
| Comparative 2 | 23 gr | 19.1 ml | 76.5 ml | 0.042 gr | 36.3 gr | 0.63 gr | 82.75% |

| | | | | | | | |
|---|---|---|---|---|---|---|---|
| M359 is the compound 3-[(5-difluoromethoxy-1-methyl-3-trifluoromethylpyrazol-4-yl)methylthio]-4,5-dihydro-5,5-dimethylisoxazole M375 is the sulfoxide impurity, 3-[(5-difluoromethoxy-1-methyl-3-trifluoromethylpyrazol-4-yl)methylsulfoxide]-4,5-dihydro-5,5-dimethylisoxazole | | | | | | | |

In these Comparative Examples 1 and 2, the average of obtained sulfoxide impurity is 48.04% and 82.75%, respectively. The amount of methanol is 0.3 liters per mole of 3-[(5-difluoromethoxy-1-methyl-3-trifluoromethylpyrazol-4-yl)methylthio]-4,5-dihydro-5,5-dimethylisoxazole in both Comparative Examples 1 and 2.

As can be seen, methanol solvent from US2023012374 cannot operate efficiently at the high concentrations of the starting material in carbonate esters.

## Claims

1. A process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, wherein the solvent comprises at least one C₁-C₂₄ carbonate ester of formula (I) and water 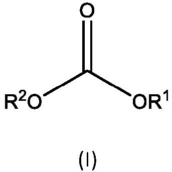 wherein
each of R¹ and R² is selected from the group consisting of C₁-C₄ alkyl; or
both R¹ and R² together form a group selected from C₁-C₄ alkylene,
and wherein the amount of the at least one C₁-C₂₄ carbonate ester ranges from 0.05 to 0.35 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole.

2. The process according to claim 1, wherein the at least one C₁-C₂₄ carbonate ester is dimethyl carbonate.

3. The process according to claim 1, wherein the at least one C₁-C₂₄ carbonate ester is diethyl carbonate.

4. The process according to claim 1, wherein R¹ and R² together are -(CH₂-CH₂)- or-(CH(CH)₃)CH₂)-.

5. The process according to claim 1, wherein the at least one C₁-C₂₄ carbonate ester is selected from the group consisting of dimethyl carbonate, diethyl carbonate, ethylene carbonate or propylene carbonate.

6. The process according to any of the previous claims, wherein the amount of the at least one C₁-C₂₄ carbonate ester of formula (I) ranges from 0.05 to 0.25 liters of C₁-C₂₄ carbonate ester per mole of 3-[(5-difluoromethoxy-1-methyl-3-trifluoromethylpyrazol-4-yl)methylthio]-4,5-dihydro-5,5-dimethylisoxazole.

7. The process according to any of the previous claims, wherein the amount of water ranges from 0.01 to 1 liters per mole of 3-[(5-difluoromethoxy-1-methyl-3-trifluoromethylpyrazol-4-yl)methylthio]-4,5-dihydro-5,5-dimethylisoxazole.

8. The process according to any of the previous claims, wherein the hydrogen peroxide is a 10 to 70 wt percent aqueous hydrogen peroxide solution.

9. The process according to any of the previous claims, wherein the metal catalyst selected from the groups 5 to 6 of the periodic table is selected from a tungsten catalyst, a molybdenum catalyst and a niobium catalyst.

10. The process according to claim 9, wherein the metal catalyst selected from the groups 5 to 6 of the periodic table is a tungsten catalyst.

11. The process according to claim 10, wherein the metal catalyst selected from the groups 5 to 6 of the periodic table is selected from the group consisting of tungstic acid, a tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture of them.

12. The process according to claim 11, wherein the metal catalyst selected from the groups 5 to 6 of the periodic table is sodium tungstate.

13. The process according to any of the previous claims, wherein the temperature of the reaction is between 30°C and 120°C.

14. A process for the preparation of pyroxasulfone according to claim 1 comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, wherein the solvent comprises dimethyl carbonate and water, wherein the amount of dimethyl carbonate ranges from 0.05 to 0.35 liters of dimethyl carbonate per mole of 3-[(5-difluoromethoxy-1-methyl-3-trifluoromethylpyrazol-4-yl)methylthio]-4,5-dihydro-5,5-dimethylisoxazole,
wherein the amount of water ranges from 0.01 to 0.8 liters per mole of 3-[(5-difluoromethoxy-1-methyl-3-trifluoromethylpyrazol-4-yl)methylthio]-4,5-dihydro-5,5-dimethylisoxazole,
and wherein the metal catalyst selected from the groups 5 to 6 of the periodic table is selected from a tungsten catalyst, a molybdenum catalyst and a niobium catalyst.

15. The process according to claim 14, wherein the amount of dimethyl carbonate ranges from 0.05 to 0.25 liters of dimethyl carbonate per mole of 3-[(5-difluoromethoxy-1-methyl-3-trifluoromethylpyrazol-4-yl)methylthio]-4,5-dihydro-5,5-dimethylisoxazole.

## Patentansprüche

1. Ein Verfahren zur Herstellung von Pyroxasulfon, das Folgendes umfasst: das Reagieren von 3-[(5-Difluormethoxy-1-methyl-3-trifluormethylpyrazol-4-yl)methylthio]-4,5-dihydro-5,5-dimethylisoxazol mit Wasserstoffperoxid in Anwesenheit eines Metallkatalysators, gewählt aus den Gruppen 5 bis 6 des Periodensystems, wobei das Lösungsmittel mindestens einen C₁-C₂₄-Carbonatester mit der Formel (I) und Wasser 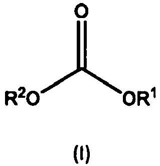 umfasst, worin
sowohl R¹ als auch R² aus der Gruppe gewählt sind, die aus C₁-C₄-Alkyl besteht, oder
beide R¹ und R² zusammen eine Gruppe bilden, die aus C₁-C₄-Alkylen gewählt ist,
und worin die Menge des mindestens einen C₁-C₂₄-Carbonatesters im Bereich von 0,05 bis 0,35 Litern C₁-C₂₄-Carbonatester pro Mol 3-[(5-Difluormethoxy-1-methyl-3-trifluormethylpyrazol-4-yl)methylthio]-4,5-dihydro-5,5-dimethylisoxazol liegt.

2. Das Verfahren gemäß Anspruch 1, worin der mindestens eine C₁-C₂₄-Carbonatester Dimethylcarbonat ist.

3. Das Verfahren gemäß Anspruch 1, worin der mindestens eine C₁-C₂₄-Carbonatester Diethylcarbonat ist.

4. Das Verfahren gemäß Anspruch 1, worin R¹ und R² zusammen -(CH₂-CH₂)- oder -(CH(CH)₃CH₂)- sind.

5. Das Verfahren gemäß Anspruch 1, worin der mindestens eine C₁-C₂₄-Carbonatester gewählt ist aus der Gruppe bestehend aus Dimethylcarbonat, Diethylcarbonat, Ethylencarbonat oder Propylencarbonat.

6. Das Verfahren gemäß einem beliebigen der obigen Ansprüche, worin die Menge des mindestens einen C₁-C₂₄-Carbonatesters mit der Formel (I) im Bereich von 0,05 bis 0,25 Litern C₁-C₂₄-Carbonatester pro Mol 3-[(5-Difluormethoxy-1-methyl-3-trifluormethylpyrazol-4-yl)methylthio]-4,5-dihydro-5,5-dimethylisoxazol liegt.

7. Das Verfahren gemäß einem beliebigen der obigen Ansprüche, worin die Menge an Wasser im Bereich von 0,01 bis 1 Liter pro Mol 3-[(5-Difluormethoxy-1-methyl-3-trifluormethylpyrazol-4-yl)methylthio]-4,5-dihydro-5,5-dimethylisoxazol liegt.

8. Das Verfahren gemäß einem beliebigen der obigen Ansprüche, worin das Wasserstoffperoxid eine 10 bis 70-prozentige (Gewichtsprozent) wässerige Wasserstoffperoxidlösung ist.

9. Das Verfahren gemäß einem beliebigen der obigen Ansprüche, worin der Metallkatalysator, der aus den Gruppen 5 bis 6 des Periodensystems gewählt ist, gewählt ist aus einem Wolframkatalysator, einem Molybdänkatalysator und einem Niobkatalysator.

10. Das Verfahren gemäß Anspruch 9, worin der Metallkatalysator, der aus den Gruppen 5 bis 6 des Periodensystems gewählt ist, ein Wolframkatalysator ist.

11. Das Verfahren gemäß Anspruch 10, worin der Metallkatalysator, der aus den Gruppen 5 bis 6 des Periodensystems gewählt ist, gewählt ist aus der Gruppe bestehend aus Wolframsäure, einem Wolframsäuresalz, metallischem Wolfram, Wolframoxid, Wolframcarbid, Wolframchlorid, Wolframbromid, Wolframsulfid, Tetrakistriwolframatophosphorsäure oder einem Salz davon, Wolframatokieselsäure oder einem Salz davon oder einer Mischung aus ihnen.

12. Das Verfahren gemäß Anspruch 11, worin der Metallkatalysator, der aus den Gruppen 5 bis 6 des Periodensystems gewählt ist, Natriumwolframat ist.

13. Das Verfahren gemäß einem beliebigen der obigen Ansprüche, worin die Temperatur der Reaktion zwischen 30°C und 120°C beträgt.

14. Ein Verfahren zur Herstellung von Pyroxasulfon gemäß Anspruch 1, das das Reagieren von 3-[(5-Difluormethoxy-1-methyl-3-trifluormethylpyrazol-4-yl)methylthio]-4,5-dihydro-5,5-dimethylisoxazol mit Wasserstoffperoxid in Anwesenheit eines Metallkatalysators umfasst, gewählt aus den Gruppen 5 bis 6 des Periodensystems, wobei das Lösungsmittel Dimethylcarbonat und Wasser umfasst, wobei die Menge an Dimethylcarbonat im Bereich von 0,05 bis 0,35 Litern Dimethylcarbonat pro Mol 3-[(5-Difluormethoxy-1-methyl-3-trifluormethylpyrazol-4-yl)methylthio]-4,5-dihydro-5,5-dimethylisoxazol liegt,
wobei die Menge an Wasser im Bereich von 0,01 bis 0,8 Litern pro Mol 3-[(5-Difluormethoxy-1-methyl-3-trifluormethylpyrazol-4-yl)methylthio]-4,5-dihydro-5,5-dimethylisoxazol liegt
und wobei der Metallkatalysator, gewählt aus den Gruppen 5 bis 6 des Periodensystems, gewählt ist aus einem Wolframkatalysator, einem Molybdänkatalysator und einem Niobkatalysator.

15. Das Verfahren gemäß Anspruch 14, worin die Menge an Dimethylcarbonat im Bereich von 0,05 bis 0,25 Litern Dimethylcarbonat pro Mol 3-[(5-Difluormethoxy-1-methyl-3-trifluormethylpyrazol-4-yl)methylthio]-4,5-dihydro-5,5-dimethylisoxazol liegt.

## Revendications

1. Procédé pour la préparation de pyroxasulfone, comprenant la réaction du 3-[(5-difluorométhoxy-1-méthyl -3-trifluorométhylpyrazol-4-yl)méthylthio]-4,5-dihydro-5,5-diméthylisoxazole avec du peroxyde d'hydrogène en présence d'un catalyseur métallique choisi parmi les groupes 5 à 6 du tableau périodique, où le solvant comprend au moins un ester de carbonate en C₁-C₂₄ de formule (I) et de l'eau 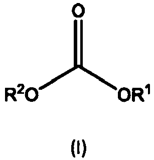 où
R¹ et R² sont chacun choisis parmi le groupe constitué d' alkyle en C₁ à C₄ ; ou
R¹ et R² forment ensemble un groupe choisi parmi alkylène en C₁ à C₄,
et où la quantité dudit au moins un ester de carbonate en C₁ à C₂₄ va de 0,05 à 0,35 litre d'ester de carbonate en C₁ à C₂₄ par mole de 3- [(5-difluorométhoxy-1-méthyl-3-trifluorométhylpyrazol-4-yl)méthylthio]-4,5-dihydro-5,5-diméthylisoxazole.

2. Procédé selon la revendication 1, où ledit au moins un ester de carbonate en C₁ à C₂₄ est le carbonate de diméthyle.

3. Procédé selon la revendication 1, où l'au moins un ester de carbonate en C₁-C₂₄ est le carbonate de diéthyle.

4. Procédé selon la revendication 1, où R¹ et R²
ensemble sont un groupe -(CH₂-CH₂)- ou -(CH(CH)₃)CH₂)-.

5. Procédé selon la revendication 1, où l'au moins un ester de carbonate en C₁-C₂₄ est choisi dans le groupe constitué par carbonate de diméthyle, carbonate de diéthyle, carbonate d'éthylène ou carbonate de propylène.

6. Procédé selon l'une quelconque des revendications précédentes, où la quantité dudit au moins un ester de carbonate en C₁-C₂₄ de formule (I) va de 0,05 à 0,25 litre d'ester de carbonate en C₁-C₂₄ par mole de 3-[(5-difluorométhoxy-1-méthyl-3-trifluorométhylpyrazol-4-yl)méthylthio]-4,5-dihydro-5,5-diméthylisoxazole.

7. Procédé selon l'une quelconque des revendications précédentes, où la quantité d'eau va de 0,01 à 1 litre par mole de 3-[(5-difluorométhoxy-1-méthyl-3-trifluorométhylpyrazol-4-yl)méthylthio]-4,5-dihydro-5,5-diméthylisoxazole.

8. Procédé selon l'une quelconque des revendications précédentes, où le peroxyde d'hydrogène est une solution aqueuse de peroxyde d'hydrogène de 10 à 70 % en poids.

9. Procédé selon l'une quelconque des revendications précédentes, où le catalyseur métallique choisi parmi les groupes 5 à 6 du tableau périodique est choisi parmi un catalyseur au tungstène, un catalyseur au molybdène et un catalyseur au niobium.

10. Procédé selon la revendication 9, où le catalyseur métallique choisi parmi les groupes 5 à 6 du tableau périodique est un catalyseur au tungstène.

11. Procédé selon la revendication 10, où le catalyseur métallique choisi parmi les groupes 5 à 6 du tableau périodique est choisi parmi le groupe constitué de l'acide tungstique, d'un sel d'acide tungstique, du tungstène métallique, de l'oxyde de tungstène, du carbure de tungstène, du chlorure de tungstène, le bromure de tungstène, le sulfure de tungstène, l'acide phosphotungstique ou un sel de celui-ci, l'acide silicotungstique ou un sel de celui-ci, ou un mélange de ceux-ci.

12. Procédé selon la revendication 11, où le catalyseur métallique choisi parmi les groupes 5 à 6 du tableau périodique est le tungstate de sodium.

13. Procédé selon l'une quelconque des revendications précédentes, où la température de la réaction est va de 30 °C à 120 °C.

14. Procédé de préparation de pyroxasulfone selon la revendication 1, comprenant la réaction du 3-[(5-difluorométhoxy-1-méthyl-3-trifluorométhylpyrazol-4yl)méthylthio]-4,5-dihydro-5,5-diméthylisoxazole avec du peroxyde d'hydrogène en présence d'un catalyseur métallique choisi parmi les groupes 5 à 6 du tableau périodique, où le solvant comprend du carbonate de diméthyle et de l'eau,
où la quantité de carbonate de diméthyle va de 0,05 à 0,35 litre de carbonate de diméthyle par mole de 3-[(5-difluorométhoxy-1-méthyl-3-trifluorométhylpyrazol-4-yl)méthylthio]-4,5-dihydro-5,5-diméthylisoxazole,
où la quantité d'eau va de 0,01 à 0,8 litres par mole de 3-[(5-difluorométhoxy-1-méthyl-3-trifluorométhylpyrazol-4-yl)méthylthio]-4,5-dihydro-5,5-diméthylisoxazole,
et où le catalyseur métallique choisi parmi les groupes 5 à 6 du tableau périodique est choisi parmi un catalyseur au tungstène, un catalyseur au molybdène et un catalyseur au niobium.

15. Procédé selon la revendication 14, où la quantité de carbonate de diméthyle va de 0,05 et 0,25 litres de carbonate de diméthyle par mole de 3-[(5-difluorométhoxy-1-méthyl-3-trifluorométhylpyrazol-4-yl)méthylthio]-4,5-dihydro-5,5-diméthylisoxazole.
